# EUROPEAN PATENT APPLICATION

(11) **EP 1 808 486 A1**
(43) Date of publication of application: **18.07.2007**
(21) Application number: 05782563.0
(22) Date of filing: 27.07.2005
(51) Int. Cl.: C12N 15/52, C12N 15/82, A01H 5/00

(54) **TRANSGENIC TOMATO PLANTS WITH ACQUIRED RESISTANCE TO PSEUDOMONAS SYRINGAE PV. TOMATO**

(30) Priority: 28.07.2004 ES 200401950
(71) Applicant: Consejo Superior de Investigaciones Cientificas, 28006 Madrid (ES); UNIVERSIDAD POLITECNICA DE VALENCIA, 46022 Valencia (ES)
(72) Inventor: VERA VERA, Pablo, Consejo Superior de Invest.Cientificas, 46022 Val (ES); COEGO GANZALEZ, Alberto, Consejo Superior de Invest.Cientificas, 46022 Val (ES)
(74) Representative: Ungria Lopez, Javier
(86) International application number: PCT/ES2005/070112
(87) International publication number: WO 2006/013226

(57) **Abstract**

The bacterial spot caused by pathogen <i> Pseudomonas syringae pv. tomato (P. s. tomato) </i> is a devastating disease affecting tomato plants. The invention shows that the mere inhibition of the expression of gene <i> Ep5C </i>, coding an extracellular cationic peroxidase is sufficient to impart marked resistance to <i> P. s. tomato </i>. Said inhibition found in tomato plants provides a resistance with no need for activation of defence pathways currently described and controlled by salicylic acid and jasmonic acid. In addition the invention shows that <i> Ep5C </i> is repressed at the transcription level in healthy plants through the formation of a stable complex including proteins and one <i> cis </i> acting element identified within the 5' region of the gene promoter. Said complex is disabled in tissue infected with <i> P. s. tomato </i> and shows maximum expression in the gene. Thus <i> Ep5C </i> gene as well as its promoter provides a new genetic asset to develop plants resisting to be bacterial spot disease.

## Description

### Scope of the invention

The invention comes within the technical sector of plants which, by means of modification via genetic engineering, acquire resistance to certain pathogens. More specifically, the invention concerns inhibition of expression of the Ep5C gene in tomato plants (genus Lycopersicon) which grants them resistance to Pseudomonas syringae pv. tomato (P. s tomato).

### State of the Art

In nature, plants are constantly attacked by pathogens causing diseases and, as a result, they have developed intricate defence mechanisms for recognising and defending themselves against a wide range of these pathogens.

One of the defensive responses most rapidly developed following recognition of a pathogen is what is known as oxidative burst, which constitutes the production of reactive oxygen intermediates (ROI), mainly superoxide (O₂) and H₂O₂, at the site where the invasion is being attempted (Apóstol et *al.,* 1989, Baker and Orlandi, 1995). The evidence that is arising suggested that oxidative burst and the related redox signalling can play a fundamental role in the integration of a diverse series of defensive responses of the plant (Grant and Loake, 2000; Loake, 2001). Following this rapid oxidative burst, two main pathways have been described via which defence genes can be activated (Dong, 1998; Kunkel and Brooks, 2002). One pathway requires salicylic acid (SA) as intermediate in the signalling, and the other requires the simultaneous perception of ethylene and jasmonic acid (JA). In contrast to the low level of understanding that exists with respect to the initial events and the genes participating in and responding to the signals generated during the oxidative burst, numerous mutations have been identified affecting the signalling via the SA and JA pathways, and some of the corresponding genes have been cloned (Hammond-Kosack and

Parker, 2003). From all these studies, a reasonable outline is starting to appear of the pathways leading from perception of the pathogen by the resistance genes to activation of the host's defences, the interconnection between them and what the contribution they make to the resistance of the plant to disease is.

Unlike what is known about how plants achieve a resistance that is both broad-spectrum and pathogen-specific, we know very little about the mechanisms and genes required for vulnerability towards certain pathogens. The vulnerability factors of the plant, which are understood here as being determinants of the host required for growth and reproduction of the pathogen and which thereby contribute to creating a favourable environment for the disease, have been widely ignored. Recently, in an effort to identify these factors, Vogel *et al.* (2002) tried to identify mutants of *Arabidopsis* which would not permit the normal growth of the pathogen of the oidium *Erysiphe cichoracearum* but which are not tied to the activation of known defensive responses such as those related to cell death, or the response pathways of SA or JA. As a result, they identified the gene *PMR6,* which codes a protein similar to the pectate lyase presumably involved in remodelling the plant cell wall. The resistance mediated by *mr6* represents a new form of resistance to the disease based on the loss of a gene required during a compatible interaction rather than the activation of known defence pathways of the host (Vogel et *al.* 2002). Also, in this context, there are classic examples of another type of host vulnerability factor such as metabolites which, functioning as signalling molecules, are capable of reprogramming in the microorganism the induction of genes required for the symbiosis or the pathogenesis (Stachel and Zambryski, 1986; Denarié et al., 1992). In general, it is assumed that vulnerability factors play important roles in growth and development and it is the pathogen which, over the course of its evolution, has become adapted to using the plant's factors for its own benefit in order to favour the disease.

### Bibliography

Agrios, G. N. (1988). Plant Pathology. Academic Press, London.
Alvarez, M.E., Pennell, R.L, Meijer, P.-J., Ishikawa, A., Dixon, R.A., and Lamb, C. (1998). Reactive oxygen intermediates mediate a systemic signal network in the establishment of plant immunity. Cell 92, 773-784.
Apostol I, Heinstein FH, Low PS. (1989). Rapid stimulation of an oxidative burst during elicitation of cultured plant cells. Role in defense and signal transduction. Plant Physiol. 90, 109-116.
Asai, T., Tena, G., Plotnikova, J., Willmann, M.R., Chiu, W.L., Gomez-Gomez, L., Bolier, T., Ausubel, F.M., and Sheen, J. (2002). MAP kinase signalling cascade in Arabidoposis innate immunity. Nature 415, 977-983.
Baker C.J., and Orlandi, E.W. (1995). Active oxygen species in plant pathogenesis. Annu. Rev. Phytopathol. 33, 299-321.
Bechtold, N., Ellis, J. and Pelletier, G. (1993). In planta Agrobacterium mediated gene transfer by infiltration of adult Arabidopsis thaliana plants. C. R. Acad. Sci. Paris Life Sci. 316,1194-1199.
Buffard, D., Breda, C., van Huystee, R.B., Asemota, 0., Pierre, M., Ha, D.B.D. and Esnault, R. (1990). Molecular Cloning of Complementary DNAs Encoding Two Cationic Peroxidases from Cultivated Peanut Cells. Proc. Natl. Acad. Sci. USA 87, 8874-8878.
Carpin, S., Crévecoeur, M., Greppin, H., and Penel, C. (1999). Molecular cloning and tissue-specific expression of an anionic peroxidase in zucchini. Plant Physiol. 120, 799-819.
Carrasco, J.L., Ancillo, G., Mayda, E., and Vera, P. (2003). A novel transcription factor involved in plant defense endowed with protein phosphatase activity. The EMBO J. 22, 3376-3384.
Chang, J.H., Tal, Y.S., Bernal, A.J., Lavelle, D.T., Staskawicz, B.J., and Michelmore, R.W. (2002). Functional analyses of the Pto resistance gene family in tomato and the identification of a minor resistance determinant in a susceptible haplotype. Mol. Plant Microbe Interact. 15, 281-291.
de Marco, A., Guzzardi, P. and Jamet, E. (1999). Isolation of Tobacco Isoperoxidases Accumulated in Cell-Suspension Culture Medium and Characterization of Activities Related to Cell Wall Metabolism. Plant Physiol. 120, 371-382.
Debener, T., H. Lehnackers, Arnold, M., and Dangl, J.L (1991). Identification and molecular mapping of a single Arabidopsis thaliana locus determining resistance to a phytopathogenic Pseudomonas syringae isolate. Plant J. 1, 289-302.
Denarié, J., Debelle, F., and Rosenberg, C. (1992). Signaling and host range variation in nodulation. Annu. Rev. Microbiol. 46, 497-531.
Dong, X. (1998). SA, JA, ethylene, and disease resistance in plants. Curr. Op. Plant Biol. 1, 316-323.
Ellul, P., Garcia-Sogo, B., Pineda, B., Rios, G., Roig, L.A., and Moreno, V. (2003). The ploidy level of transgenic plants in Agrobacterium-mediated transformation of tomato cotyledons (Lycopersicon esculentum L. Mill.) is genotype and procedure dependent. Theor. Appl. Genet. 106, 231-238.
Foster, R., Gasch, A., Kay, S. and Chua, N.-H. (1992). Analysis of protein-DNA interactions. In Koncz, C., Chua, N.-H. and Schell, J. (eds.), Methods ín Arabídopsis Research. World Scientific Publishing, Singapore, pp. 378-392.
Gadea, J., Mayda. E., Conejero, V. and Vera, P. (1996). Characterization of defense-related genes ectopically expressed in viroid-infected tomato plants. Mol. Plant Microbe lnterac. 9, 409-415.
Gardan, L., Shafik, H., Belouin, S., Broch, R., Grimont, F., and Grimont, P.A. (1999). DNA relatedness among the pathovars of Pseudomonas syringae and description of Pseudomonas tremae sp. nov. and Pseudomonas cannabina sp. nov. (ex Sutic and Dowson 1959). Int. J. Syst. Bacteriol. 49, 469-478.
Grant, J.J., and Loake, G. J. (2000). Role of reactive oxygen intermediates and cognate redox signaling in disease resistance. Plant Physiol, 124, 21-29.
Greenberg, J.T., and Vinatzer, B.A. (2003). Identifying type III effectors of plant pathogens and analysing their interaction with plant celis. Curr. Opin. Microbiol. 6, 20-28.
Glazebrook, J., Rogers, E.E., and Ausubel, F.M. (1996). Isolation of Arabidopsis mutants with enhanced disease susceptibility by direct screening. Genetics 143, 973-982.
Gubbler, F., Raventos, D., Keys, M., Watts, R., Mundy, J., and Jacobsen, J.V. (1999). Target genes and regulatory domains of the GAMYB transcriptional activator in cereal aleurone. Plant J. 17, 1-9. Hammond-Kosack, K. E., and Parker, J. E. (2003). Deciphering plant-pathogen communication: fresh perspectives for molecular resistance breeding. Curr. Op. Biotechnol. 14,177-193.
Hiraga, S., Sasaki, K., Ito, H., Ohashi, Y., and Matsui, H. (2001). A Large Family of Class lIl Plant Peroxidases. Plant Cell Physiol. 42, 462 - 468.
Jabs, T., Dietrich, R.A., and Dangl, J.L. (1996). Initiation of runaway cell death in an Arabidopsis mutant by extracellular superoxide. Science 273, 1853-1856.
Jefferson, R.A. (1987). Assaying chimeric genes in plants: The GUS gene fusion system. Plant Mol. Biol. Reporter 5, 387-405.
Jones, J.B., Jones, J.P., Stall, R.E., and Zitter, T.A. (1991). Compendium of tomato diseases. St. Paul: APS Press.
Jordá, L, Coego, A., Conejero, V., and Vera, P. (1999). A genomic cluster containing four differentially regulated subtilisin-like processing protease genes is in tomato plants. J. Biol. Chem. 274, 2360-2365.
Kim, Y.J., Lin, N., and Martin, G.B. (2002). Two distinct Pseudomonas effector proteins interact with the Pto kinase and activate plant immunity. Cell 109, 589-598.
KunkeI, B.N. and Brooks, D.N. (2002). Cross talk between signaling pathways in pathogen defense. Curr. Op. Plant Biol. 5, 325-331.
Kunkel, T.A., Roberts, J.D., Zakour, R.A. (1987). Rapid and efficient site specific mutagenesis without phenotypic selection. Methods Enzymol. 154, 367-381.
Lamb, C. and Dixon, R.A. (1997). The oxidative burst in plant disease resistance. Ann. Rev. Plant Physiol. Plant Mol. Biol. 48, 251-275.
Loake, G.J., Faktor, 0., Lamb, C.J. and Dixon, R.A. (1992). Combination of H-box [CCTACC(N)7GT] and G-box (CACGTG) cis elements is necessary for feed-forward stimulation of a chalcone synthase promoter by the phenylpropanoid-pathway intermediate p-coumaric. Proc. Natl. Acad. Sci. USA 89, 9230-9234.
Loake, G.J. (2001). Plant cell death: Unmasking the gatekeepers. Current Biology 11, 1028-1031.
Martin, G.B., Brommonschenkel, S.H., Chunwongse, J., Frary, A., Ganal, M.W., Spivey, R., Wu, T., Earle, E.D., and Tanksley, S.D. (1993). Map-based cloning of a protein kinase gene conferring disease resistance in tomato. Science 262,1432-1436.
Mayda, E., Tornero., P., Conejero, V., and Vera, P. (1999). A tomato homeobox gene (HD-Zip) is involved in limiting the spread of programmed cell death. Plant J. 20, 591-600.
Mayda, E., Marques, C., Conejero, V., and Vera, P. (2000a). Expression of a pathogen-induced gene can be mimicked by auxin insensitivity. Mol. Plant-Microbe Interact. 13, 23-31.
Mayda, E., Mauch-Mani, B., and Vera, P. (2000b). The Arabidopsis dth9 mutant is compromised in systemic acquired resistance without affecting SA-dependent responses. Plant Cell. 12, 2119-2128.
Mysore, K.S., Crasta, O.R., Tuori, R.P., Folkerts, 0., Swirsky, P.B., and Martin, G.B. (2002). Comprehensive transcript profiling of Pto- and Prf-mediated host defense response to infection by Pseudomonas synrigae pv. syringae. PIant J. 32, 299-315.
Price, N.J., Pinheiro, C., Soares, C.M., Ashford, D.A., Ricardo, C.P., and Jackson, P.A. (2003). A Biochemical and Molecular Characterization of LEP1, an Extensin Peroxidase from Lupin . J. Biol. Chem. 278, 41389 - 41399.
Stachel, S.E., and Zambryski, P.C. (1986) . VirA and VírG control the plant-induced activation of the T-DNA transfer process of A. tumafaciens. Cell 46, 325-333. Sutherland, M.W. (1991). The generation of oxygen radicals during host plant responses to infection. Physiol. Mol. Plant Pathol. 39, 79-93.
Tang, X., Frederick, R., Zhou, J., Halterman, D., Jia, Y. and Martin, G.B. (1996). Initiation of plant disease resistance by physical interaction of AvrPto and Pto kinase. Science 274, 2060-2063.
Tornero, P., Gadea, J., Conejero, V. and Vera, P. (1997). Two PR-1 genes from tomato are differentially regulated and reveal a novel mode of expression for a pathogenesis-related gene during the hypersensitive response and development Mol. Plant-Microbe Interac. 5, 624-634.
Vorwork, S., Somerville, S. and Somerville, C. (2004). The role of plant cell polysaccharide composition in disease resistance. Trends Plant Sci. 9, 203-209.
Whalen, M.C., Innes, R.W., Bent, A.F., and Staskawicz, B.J. (1991). Identification of Pseudomonas syringae pathogens of Arabidopsis and a bacterial locus determining avirulence on both Arabidopsis and soybean. Plant Cell 3, 49-59.
Whetten, R., and Sederoff, R. (1995). Lignin biosynthesis. Plant Cell 7, 1001-1013.
Zhao, Y., Thilmony, R., Bender, C.L., Schaller, A, He, S.Y. and Howe, G.A. (2003): Virulence systems of Pseudomonas syringae pv. tomato promote bacterial speck disease in tomato by targeting the jasmonate signal pathway. Plant J. 36, 485-499.

### Brief description of the invention

The invention consists of the characterisation of *Ep5C* and its promoter (SEQ ID NO: 1), a tomato plant gene which encodes an extracellular peroxidase enzyme whose expression is quickly derepressed by the H₂O₂ generated in the course of plant-pathogen interaction. The invention shows that, when the inducible expression of *Ep5C* is inhibited in transgenic plants with antisense *Ep5C,* a resistance response to the disease takes place in those transgenic plants towards *P*. *syringae* pv. tomato, the causal agent of spot disease. These results show that Ep5C represents a new plant vulnerability factor, which permits the genetic modification of tomato varieties, in such a way that said gene is not expressed, conferring pest resistance.

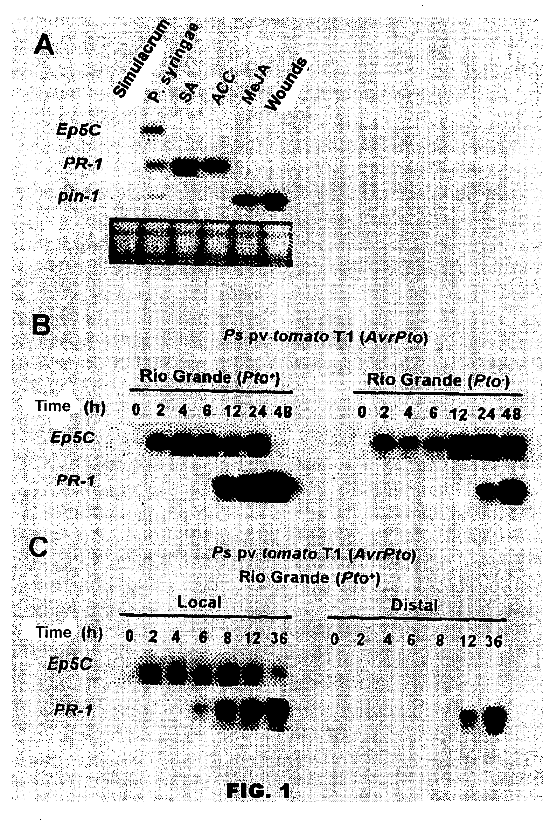

In the invention *Ep5C* is characterised, a tomato plant gene which, when inhibited, makes the plant more resistant to *P. syringae* pv. tomato, the causal agent of spot disease. *Ep5C* codes an apoplastic protein which shares a significant similarity in sequence with plant cationic peroxidases (Gadea, *wet al.,* 1997, Higara et al., 2001). There are numerous isozymes of peroxidases in plants, most of them being located extracellularly and in vacuoles (Carpin et *al.,* 1999), and their genes can be induced by a large variety of stimuli including wound formation (Bernards et *al.* 1999), pathogens (Lamb and Dixon, 1997) and also during protoplast regeneration and in different states of development of the plant (de Marco et al. 1999). In particular, it has been proposed that some cationic peroxidases act in the polymerisation of phenolic monomers to form lignin and suberin or for mediating in the crosslinking of polysaccharides and proteins with cell walls, and therefore playing an important role in the building of cells walls and the plasticity associated with them (Whetten and Sederoff, 1995). In general, peroxidases are involved in all these biological processes by catalysing a lot of oxidation-reduction reactions in the presence of H₂O₂, which functions as an oxidising substrate. It is interesting to point out that H₂O₂ plays a dual role as far as *Ep5C* is concerned: it functions as a substrate for the encoded peroxidase enzyme and also as an inductor signal which starts up the characteristic activation of *Ep5C* transcription following the perception of the pathogenic bacterium P. s. tomato. In this regard *Ep5C* expression is rapidly induced in the course of both compatible and incompatible interactions with P. s. tomato and the induction takes place locally and not in non-inoculated distant tissues (Figures 1, 2 and 4). This pattern of gene expression is consistent with the early and local generation of H₂O₂ by the plant cell once the presence of the live pathogenic bacterium has been perceived (Grant and Loake, 2000).

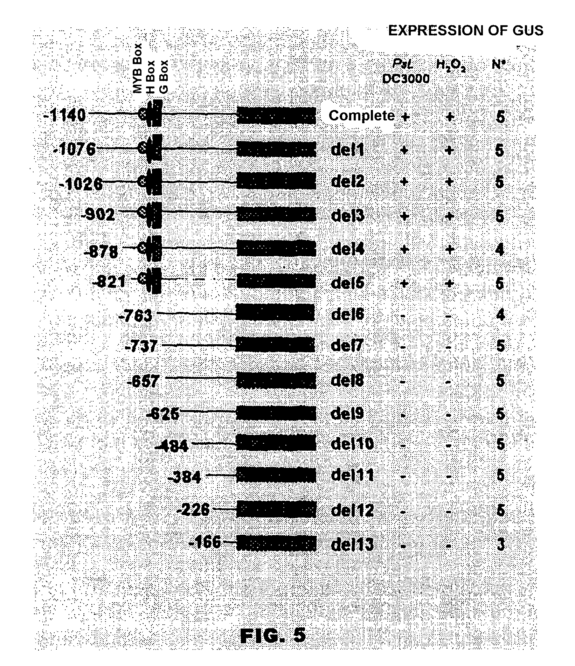

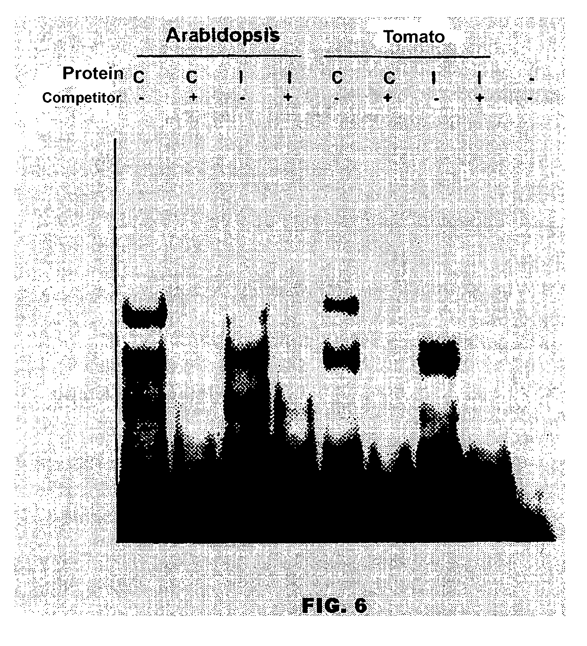

The functional analysis of *Ep5C* promoter in transgenic plants has revealed that the necessary sequences for the induction of the gene is found between positions -821 and -763 (Figure 5) or between nucleotides 320 to 378 of the SEQ ID NO1. Within that region of the *Ep5C* promoter, an element has been identified which acts in *cis* which, under rest conditions, when the gene is not expressed, is joined in a specific way to two different protein factors from the leaves (Figure 6). The formation of one of these DNA-protein complexes is sensitive, both in tomato and in *Arabidopsis,* to the presence of P. s. tomato, with loss of the union and activation of *Ep5C* gene taking place simultaneously.

Another aspect of this invention reveals that the DNA-protein complex differentially regulated and identified here is acting by repressing the *Ep5C* gene transcription. The target sequence of this complex is a short region of 51 bp known as R1 (between positions 320 to 370 of SEQ ID NO: 1) where three well-characterised plant elements which act in cis can be identified: the conserved element AAATTGTTA (MYB box; from 335 to 343 of SEQ ID NO1), characteristic of some genes regulated by myb (Gubbler et *al*., 1999), a sequence similar to an H box (GGTAGG, from 344 to 349 of SEQ ID NO1 and a G box sequence (CACGTG, from 354 to 359 of SEQ ID NO1), similar to those conserved in some genes of the phenylpropanoids pathway (Loake et al., 1992). The involvement of R1 element of 51 bp identified in the response capacity of *Ep5C* promoter is also backed up by experiments in transgenic plants of *Arabidopsis* in which the presence of this sequence is fundamental for the response capacity both with infection by P. s. tomato and with attack from H₂O₂ (see Figure 5, *del5* deletion against *del6* deletion). Nevertheless, in *del6* deletion and subsequent downstream deletion, where the R1 element is no longer present and in which the union of the supposed repressor complex would have to be impeded, the expression of Ep5C gene is not randomly regulated and induction of the expression is disabled by the bacterial infection or by attack from H₂O₂.

Another interesting aspect of the invention and the role of *Ep5C* during plant-pathogen interaction is the discovery that absence of expression of this gene, as in antisense transgenic tomato plants, confers great resistance to *P. s*. *tomato. Pseudomonas syringae* is an extracellular pathogen which infects a wide variety of plants (Gardan et al., 1999) and, in contrast to our profound understanding of the resistance mechanism to bacterial spot disease existing in the State of the Art (Kim et al., 2002; Mysore et al., 2002; Tang et al., 1996), very little is known about the molecular bases of host vulnerability to virulent strains of *P. syringae* and/or the mechanism used by this pathogen to provoke the disease (Greenberg and

Vinatzer, 2003). It is widely recognised that plants, even in the absence of a gene with gene interaction, can develop an active defence response which partially limits virulent *P*. *syringae* (Glazebrook et al., 1996). It is not clear what triggers this resistance, but it could be the perception of wounds by the plant, the capacity to detoxify the polyketide coronatine synthesised by the bacterium (Zhao et *al.,* 2003), the recognition of molecular patterns associated with the pathogen (Asai et al., 2002), a weak recognition of type III effectors injected inside the plant cell (Chang et *al.,* 2002) or even combinations of these. In this scenario, the Ep5C requirement of a correct and compatible interaction to take place between *P. s*. *tomato* and the tomato plants can only be understood from two perspectives: either Ep5C is required for facilitating bacterial growth or loss of Ep5C in some way results in the activation of defences in the host which halt the bacterial infection. As the increase in expression of defence markers (for example, *PR-1, P69, pin-1)* is not observed in uninfected antisense plants, the resistance of antisense Ep5C does not appear to be mediated by constitutive activation of the defensive pathway dependent on SA or dependent on JA. Similarly, as the induction levels of expression of the same genes related to defence were no greater than those of the wild type plants following infection with P. s. *tomato,* the observed resistance seems not to be caused by a hyperactivation of these pathways in antisense plants. All these observations leave resistance mediated by antisense *Ep5C* outside of the resistance mechanisms previously described which are characterised by boosting the expression of genes related to defence (Hammond-Kosack and

Peter, 2003). So, the invention concludes that there exists a gene which, when correctly expressed, either facilitates the growth of P. s. tomato or, alternatively, it suppresses an unknown basal defensive pathway of the plant. These considerations are consistent with Ep5C functioning as a host factor of the tomato plants required for vulnerability to infection with P. s. *tomato.*

Ep5C represents a new factor of the plant that is required for the growth of *P. syringae,* and reducing its accumulation produces a reduction in pathogen growth. Such a reduction in pathogen growth is a new form of resistance to disease based on loss of the capacity of the host to support the pathogen independently of the resistance mediated by R genes or activation of the host's defences mediated by the signalling molecules SA or JA.

To summarise, the resistance mediated by *Ep5C* can be considered as a special form of resistance to the disease against *Pseudomonas syringae,* based on the loss of a host vulnerability gene required by the pathogen for growth and development. The antisense transgenic plants of the invention resistant to the disease caused by Pseudomonas syringae pv. tomato (P. s. tomato) do not constitute plant varieties of the genus Lycopersicon. The present invention does not apply to any particular variety but instead has application to higher taxa.

Although the invention gives details of a non-limiting example of embodiment thereof, the inhibition of Ep5C gene by means of an antisense construction, the inhibition of the gene expression can be carried out by means of any other alternative technique, such as: iRNA (interference RNA), introduction of foreign DNA in the coding region (knock-out), homologous recombination, etc.

Likewise, the use of the promoter of the invention in the regulation of gene expression in other genes different from Ep5C is also evident, due to which its use in the repression of Ep5C expression also must not be regarded as limiting. This use particularly corresponds to claim 10 and is highlighted in experiments carried out with the GUS gene, a gene from a microorganism, and its expression regulated by the promoter of the invention in Arabidopsis thaliana, a plant species.

### Detailed description of the invention

A preferred embodiment of the invention corresponds to claim 1. The inhibition strategy of Ep5C gene consisted in producing transgenic plants with inhibited expression of that gene. To achieve this, antisense transgenic plants were produced.

An antisense transgenic plant is a plant that expresses a RNA, or part thereof, but in the opposite/inverted sense to its normal translation frame. In this way, when said antisense RNA is present in the transgenic cell it will complement the endogenous RNA and this provokes an artefact in the endogene structure that prevents its translation into protein or activates its degradation, or something similar. The ultimate result is that the endogenous gene does not translate into protein and it is therefore as if the expression of the endogenous gene had been inhibited. The sequence used to generate the expression cassette of transforming plants corresponds to 309 nucleotides from the coding sequence of the Ep5C gene cloned under the 35S promoter but in the inverted sense for generating *in vivo* a fragment of antisense RNA complementary to that of the endogenous gene.

The invention has non-limited applications to transgenic plants of the Solanaceae family (potato, tomato, peppers, tobacco, etc.), particularly to the genus Lycopersicon (tomato).

### Example 1

**Generation and analysis of transgenic plants.** To generate the construct with the normal transcription sense, a fragment of 1150 bp (almost the complete length) was cut from *PCEVI16* with *Smal* and *Kpnl* and bonded between the 35S promoter of cauliflower mosaic virus (CaMV) and the NOS termination sequence of the pBI121 plasmid (Clontech) resulting in *p35S-Ep5Csense.* pCEV16 is the name of a commercial pBlusescript plasmid in which a cDNA is housed numbered as 16 and which encodes a peroxidase type protein. This cDNA was initially isolated in a cDNAs library made from tomato plants. It served as a probe for isolating a genomic clone of tomato that we called Ep5C and which encodes the peroxidase protein called Ep5C. The cDNA contained in pCEV16 or the similar DNA derived from the genomic Ep5C clone was amplified and digested with restriction enzymes for its cloning in a binary transformation vector in order to generate transgenic plants.

For the construction of antisense *Ep5C,* a fragment containing the 309 nucleotides closest to the 3' end of pCEVI16 was amplified by PCR using Pfu DNA polymerases (Stratagene, San Diego, California) and it was subcloned in the antisense direction in the binary vector pBI121 (Clontech) under control of the 35S constitutive promoter of CaMV in order to give *p35S-Ep5Cantisense.* The constructions were transferred to A. *tumefaciens* LB4404 and were used for transforming UC82B tomato plants as described earlier (Mayda et al., 1999, 2000) using the method of McCormick et *al.* (1986). The resulting transformants were selected for their resistance to kanamycin. A total of 25 primary transformants were produced, 15 with *p35S-Ep5Csense* and 10 with *p35S-Ep5Cantisense.* Transgene inheritance was checked by means of amplification of the transgene by PCR and estimating the percentage of R2 plantules that grew on agar with kanamycin. This latter assay permitted the plants to be provisionally designated as azygotic, hemizygotic or homozygotic. All experiments were conducted with plantules that were homozygotic for each of the transgenes considered. The complete length *Ep5C* promoter of 1140 pb and the 5'deletions of this promoter were generated by PCR using specific sequence primers in which the synthetic restriction sites were incorporated by means of mutagenesis targeted to the site (Kunkel et *al.,* 1987). The corresponding DNA fragments were cloned upstream with respect to -glucuronidase gene in PBI101.1 (Jefferson et al., 1987) resulting in transcriptional fusions with the coding sequence of the GUS indicator gene. The resulting fusions were verified by nucleotide sequence analysis using specific primers for the promoter regions. The plasmids were then transferred to A. *tumefaciens* C58 and used for transforming *Arabidopsis thaliana* Col-0 as has been described (Bechtold et al., 1993). The activity of GUS was tested in the transformants by a fluorimetric assay or by an *in situ* assay using the colorigenic substrate X-gluc (Jefferson, 1987). F₁ seeds were made to germinate on a selective medium with agar MS which contained kanamycin and the transgenic plantules were then transferred to soil.

### Example 2:

### Inoculations with bacteria, determination of cfu and chemical treatments

Strains of *P. syringae* were made to grow during an entire night in King medium B which contained antibiotics, they were washed twice, resuspended and diluted with 10 mM MgCl₂ to the indicated densities. The bacteria suspensions were infiltrated on the abaxial surface of the leaves using a 1 ml syringe without needle as described for tomato (Mayda et al., 1999) and for *Arabidopsis* (Mayda et al., 2000b).

At the appropriate time-points three independent foliar discs (each of 1 cm²) were taken from infiltrated leaves of P. s. tomato (2.5 x 10⁵ cfu/ml). The discs were randomly divided into groups of three and were mashed in 10 mM MgC1₂. The density of bacterial populations was determined by serial dilution plating and count of colony forming units in King medium B supplemented with rifampicin (50 m/ml) at 28 °C.

Leaves were inoculated with a superoxide generator system (xanthine (5 mM) and xanthine oxidase (0.05 U/ml) (X-XO)) or with a H₂O₂ generator system (D-glucose 2.5 mM and glucose oxidase (2.5 U/ml) (G-GO)) freshly prepared in a 20 mM sodium phosphate buffer (pH 6.5) as has been previously described (Mayda et *al.,* 1999). At the appropriate moment, 600 U/ml of radish SOD (Sigma) or 100 U/ml of catalase (Sigma) were included in the reaction.

### Example 3:

**Electrophoretic mobility shift assays.** Extracts from complete foliar cells and retardation assays in gel were carried out as described previously (Foster et al., 1992; Carrasco et al., 2003). As a probe, a double strand oligonucleotide (R1) was radioactively dyed filling *Sall* extensions at 5' and 3' ends of the Klenow fragment of the *E. coli* DNA polymerase I. The binding reactions contained 8 fmols of probe and 20 g of foliar protein in Hepes 20 mM-KOH of pH 7.6, 40 mM KCl, 0.5 mM EDTA, 1 mM DTT, 200 g of BSA, 10% glycerol in the presence of 200 ng of poly(di-dC).poly(di-DC), and were incubated at room temperature for 15 minutes. DNA-protein complexes were separated from free DNA probes by electrophoresis in native gels of 6% polyacrylamide (38:2) made in TBE 0.5x at 4°C.

### Example 4:

**Gene library tracking and DNA sequence analysis.** A tomato genome DNA gene library constructed in -EMBL3 (Clontech) was tracked with a cDNA probe for Ep5C, radioactively dyed, and positive clones were purified and characterised as has been described (Tomero *et al*., 1997). DNA sequencing was carried out in a DNA ABI PRISM 377 sequencer (Perkin-Elmer, Foster City, California). The computer assisted analysis of the DNA sequence was performed using the software package from Genetics Computer Group (GCG) of the University of Wisconsin (Genetics Computer Group, Inc., Madison, Wisconsin).

### Example 5:

**Analysis with RNA transfer:** RNA from tomato leaves was purified as has been described (Jordá et *al.,* 1999) and 15 g of total RNA were submitted to electrophoresis in 1% agar gels containing formaldehyde and were transferred to Nytran membranes (Schleicher & Schuell). It was verified that equal quantities of RNA had been charged by means of gel staining with ethidium bromide prior to transfer to the membrane. Radioactively dyed probes were prepared by random initiation using T7 polymerase (Pharmacia). The hybridisation and washing conditions of the filters were as has been described (Jordá et al., 1999).

### Example 6:

**Production of recombinant Ep5C and generation of polyclonal antiserum.** Ep5C coding sequence was amplified by PCR using *Pfu* DNA polymerase and it was subcloned in the pGEX vector (Amersham Biosciences) for its expression in *E. coli* as a fusion protein with glutation-S-transferase (GST). The recombinant protein was affinity purified via a column of glutation-Sefarose 4B (Amersham Biosciences) respectively, following the manufacturer's instructions. Fifty micrograms of purified fusion protein were suspended in Freund complete adjuvant and were injected via subcutaneous route into a New Zealand white rabbit. Booster injections were given at 2 week intervals for 3 months. The rabbit was then made to bleed and serum was collected.

### Example 7:

**Western type transfer.** Raw foliar extract of tomato leaves were prepared in HEPES 20 mM-KOH of pH 7.6, 40 mM KCl, 0.5 mM EDTA, 1 mM DTT, 0.5 mM PMSF or directly in loading buffer containing SDS. The PAGE in SDS and the Western type transfer were carried out in accordance with standardised procedures. A 1:500 dilution of anti-Ep5C antiserum was used.

### Example 8:

### Expression pattern of Ep5C in tomato plants

Isolation of a set of cDNA clones which represent regulating genes in order to increase their expression in diseased tissues of the tomato plant has been previously described (Gadea *et al.,* 1997). While some of these genes have been characterised in detail (Mayda et *al.,* 2000a, 2000b, Carrasco et *al.,* 2003), others remain unexplored.

One of these latter genes, referred to here as *Ep5C,* which corresponds to one of the cDNA clones previously described (for example, pCEVI-16; Gadea et *al.,* 1997) encodes a cationic peroxidase of 32 kDa which has a high homology with peroxidases secreted from peanut PNC2 (Buffard et al., 1990). In the present invention detailed studies have been conducted with *Ep5C* because it develops an interesting gene expression pattern following attack from *Pseudomonas syringae* pv. *tomato* the causal agent of bacterial spot disease. In fact, expression of the *Ep5C* gene is induced in tomato leaves following inoculation of the bacterium in a manner similar to that observed in the case of induction of the classic gene expression *PR-1* (Figure 1A). Nevertheless, no induction of Ep5C was observed when signalling molecules, such as salicylic acid (SA), 1-aminocyclopropane-1-carboxylate (ACC, the precursor of ethylene), methyl-jasmonate (MeJ) which mediate the induction of defences, were infiltrated into foliar tissue, not even when wounds were provoked, while the corresponding marker genes used as positive controls (for example, *PR-1* in the case of SA or ACC, or pin-1 for MeJ and wound formation) were clearly induced in treated leaves (Figure 1A). In order to understand in further detail how the expression of *Ep5C* gene could be regulated in the course of compatible and incompatible plant-pathogen interactions, and likewise in the course of a normal HR response, we inoculated tomato plants, either resistant (Pto⁺) or vulnerable (Pto⁻) with *P.* s. *tomato* and with or without the avirulence gene *avrPto* (Martin et *al.,* 1993). Following inoculation with *P.* s. *tomato-avrPto,* there was a high accumulation level of *Ep5C* transcripts 2 hours postinoculation (h.p.i.) in both Pto and pto plants (Figure 1B), but not in plants in which inoculation was simulated. Surprisingly, massive accumulation of *Ep5C* transcripts was merely transitory in the incompatible interaction and no detectable levels of mRNA accumulation remained at 48 h.p.i. On the contrary, during the course of compatible interaction, induction of *Ep5C* expression persisted at 48 h.p.i. (Figure 1B) and for an even greater length of time.

This abrupt induction of *Ep5C* expression gene works on a local basis, since it occurs only in tissues that are close to the inoculation site and not in non-inoculated tissues of the other half of the same leaf as shown in Figure 1C. In the distal tissues with respect to the inoculation site, *Ep5C* expression is not induced while the activation of the marker gene *PR-1* expression is stimulated (Figure 1C). These results reveal that a signal generated locally during the course of plant-bacterium interaction triggers the characteristic *Ep5C* activation. So, the early and local induction of *Ep5C* together with the lack of effect of SA, JA or ET on its expression rules out any trivial explanation for the signal and the mechanism which mediates *Ep5C* induction during the plant-bacterium interaction considered.

### Example 9:

### Ep5C induction by H₂O₂

Production of extracellular superoxide and subsequent generation of H₂O₂ are early signals generated during the oxidative burst triggered by both compatible and incompatible interactions (Sutherland, 1991). These signals are critical for initiating a hypersensitive response cell death programme (HR, Jabs et al., 1991) and also for the induction of protector genes (for example, GST (Alvarez *et al*., 1998) in the plant.

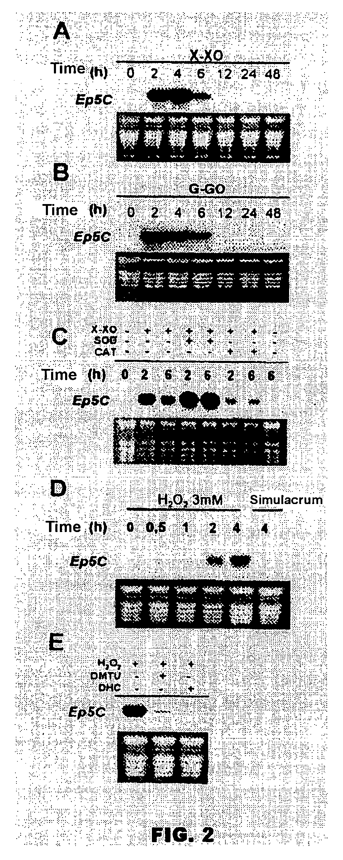

For that reason, we focused on whether the local production of extracellular superoxide (by means of xanthine and xanthine oxidase (X-XO) infiltration in the intercellular spaces) or H₂O₂ (by means of glucose and glucose oxidase (G-GO) infiltration in the intercellular spaces) would trigger *Ep5C* gene expression. The results of Figure 2A-B indicate that both treatments were capable of triggering the induction of *Ep5C* expression, transitorily and with a similar magnitude. This suggests that the characteristic pattern of expression of this gene following inoculation of P. s. *tomato* can be modulated either by superoxide or by H₂O₂ generated in the apoplast following plant-pathogen interactions (Hammond-Kosack and

Jones, 1996). Given that the extracellular generation of superoxide during pathogen interactions is quickly converted to H₂O₂ by the action of extracellular superoxide dismutase (SOD) (Sutherland, 1991), we considered the possibility that the real inducer of *Ep5C* expression might be H₂O₂ and not the superoxide. In the experiments shown in Figure 2C, the induced *Ep5C* expression as a result of the superoxide generated in the intercellular spaces after infiltrating a reaction mixture with X-XO was inhibited when the enzyme catalase (which degrades H₂O₂) was simultaneously infiltrated with the reaction mixture with X-XO. Reciprocally, when the enzyme SOD (which converts superoxide to H₂O₂) was co-infiltrated with the reaction mixture with X-XO, *Ep5C* expression was superinduced with respect to the induction level observed when only the reaction mixture with X-XO was used. As a complementary test, we checked to see if H₂O₂, when infiltrated in the intercellular spaces, is capable of promoting *Ep5C* expression. Figure 2D shows that H₂O₂ *per* se is capable of promoting *Ep5C* expression. Moreover, dimethylthiourea (DMTU), which acts as a trap for H₂O₂, and also the methyl ester of 2,5-dihydroxycinamic acid (DHC), which acts as a trap for H₂O₂ mediated by peroxidases, is capable of blocking *Ep5C* induction by exogenous H₂O₂ (Figure 2E). All these results prove that H₂O₂ is the signal that starts up the characteristic expression of *Ep5C.*

### Example 10:

### Analysis of Ep5C gene expression in transgenic plants of Arabidopsis containing a promoter-GUS fusion

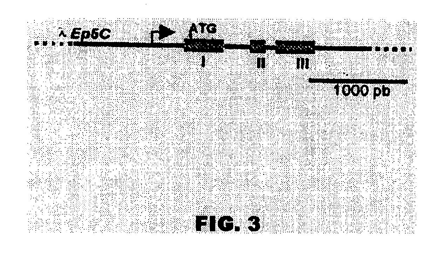

In order to achieve further information on how *Ep5C* is organised in the genome, cDNA was used as a probe in order to isolate the structural gene from a -EMBL3 gene library of tomato genomic DNA. This tracking permitted the isolation of a genomic clone with an insert of 33 kb which was sequenced and characterised. The alignment of the genome sequences and cDNA revealed the presence of 2 introns in the transcribed region (SEQ ID NO: 1 and Fig. 3). Also, analysis by Southern type transfer of tomato DNA digested with different enzymes indicates that *Ep5C* exists as a gene with a single copy per haploid genome.

The examination of conserved motifs in the 5'sequence of the *Ep5C* gene promoter, revealed the presence of a conserved element AAATTGTTA which acts in cis, between positions 335 and 343 of the SEQ ID N01, which is characteristic of some genes regulated by myb (Gubbler et al., 1999). Adjacent to this motif is the presence of a sequence similar to an H box (GGTAGG; from 344 to 349 of SEQ ID NO1) and a G box (from 354 to 359 of SEQ ID N01) similar to those conserved in some genes of the phenylpropanoid pathway (Loake et al., 1992).

In order to delve further into our understanding of the mode of gene activation characteristic of *Ep5C,* 1140 bp of the5' flanking promoter were fused with *GUS* indicator gene. This fragment was introduced in *Arabidopsis* plants by transformation with *A. tumefaciens,* and the transgenic plants were used for studying the regulation of *Ep5C* transcription. Various transgenic lines were selected containing a single insert of the *Ep5C-GUS* construction and expression of *GUS* was tested in them at tissue level.

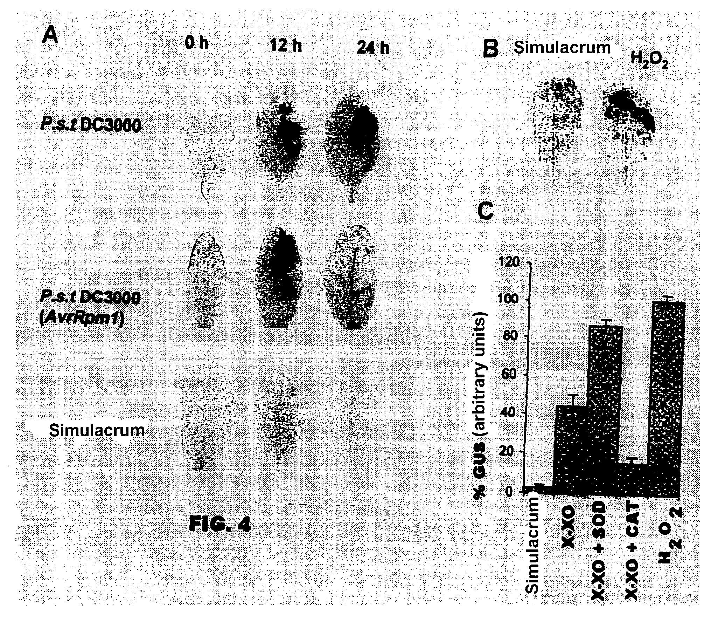

None of the selected transgenic lines showed any constitutive expression of *Ep5C-GUS* but in its place they showed induction of GUS expression after attack with different signals and in a manner which reproduced the data derived from the analysis with Northern type transfer of tomato plants shown earlier. In fact, GUS expression was induced locally following inoculation of *Pseudomonas syringae* pv. tomato DC3000 (P.s.t. DC3000; Whalem et *al.,* 1991) which gives rise to a compatible interaction in the Col-0 ecotype used for generating transgenics (Figure 4A). In a similar way, inoculation of the same transgenic lines of *Arabidopsis* with Pst DC3000 which carried the avirulence gene *avrRpm1* (Debener et *al.,* 1991) generates an incompatible interaction which also gives rise to local induction of GUS activity at the site of inoculation with bacteria. Nevertheless, this last induction of GUS expression was not lasting due to the fact that it disappeared shortly after the HR programme was executed and the plant halted the bacterium advance (Figure 4A). No expression was observed in plants in which inoculation was simulated (Figure 4A). So, results are compatible with what has been observed in tomato plants (Figure 1) and they help to interpret the fact that the observed activation of *Ep5C* gene transcription takes in a local manner only, being induced by Pseudomonas *syringae* and appearing independently of whether the bacterium used is virulent or avirulent.

Additionally, experiments aimed at studying the effect of ROI species on *Ep5C-GUS* expression in *Arabidopsis* revealed that H₂O₂ also triggers gene induction. Both direct infiltration of a solution of H₂O₂ in the intercellular spaces of *Arabidopsis* leaves (Figure 4B) and its direct generation starting from a reaction mixture with G-GO are capable of triggering local induction of GUS activity controlled by the *Ep5C* promoter. Moreover, the combinations of reaction mixtures with X-XO in which catalase or SOD are co-infiltrated in the intercellular spaces of leaves of transgenic Arabidopsis (Figure 4C) reproduce the data derived from tomato plants (Figure 2) and they again highlight the interpretation that H₂O₂ is the signalling molecule which triggers *Ep5C* expression. So, these studies confirm that the information relating to all main aspects of the *Ep5C* gene induction is contained within the first 1140 bp of the 5' region of the promoter.

### Example 11: Identification of a specific site for binding to proteins within the region of the Ep5C promoter

As a first step to define the elements of the DNA sequence which could be important for mediating the induction of *Ep5C* gene expression, a series of mutants were created with unidirectional deletions in the promoter. The original complete length promoter 1140 bp was modified as described above. The resulting constructions with - glucuronidase were introduced in Arabidopsis by stable transformation mediated by *Agrobacterium.* The detection of GUS activity was carried out in samples of leaves from these transgenic plants, before and after inoculation with P.s. tomato DC300 and after infiltration with a 3 mM solution of H₂O₂. Via a series of unidirectional deletions in 5' shown in Figure 5, we observed that 821 bp of the promoter sequence (construction de15) were sufficient for maintaining a complete response capacity to both inoculation with bacteria and treatment with H₂O₂, while the 763 bp of the promoter sequence (construction del 6) were not. Gene induction was never able to be recovered in any of the successive deletions downstream shown in Figure 5. These results therefore indicate that positions 320 to 370 of SEQ ID N01 are fundamental for *Ep5C* gene induction. It is within this short segment of DNA of 63 bp where the MYB, G and H boxes described above are centred. In order to check any potential protein-DNA interaction within this region of the promoter, we carried out electrophoretic mobility shift assays (EMSA) with extracts of proteins derived from control tomato leaves and leaves infected with P.s. tomato. As a probe a double strand oligonucleotide was used which covered 51 bp (320 to 370 of SEQ ID NO1) of the minimum *Ep5C* promoter identified above, and known as R1. When the radioactively dyed R1 probe was incubated with a protein extract prepared starting from control tomato plant leaves, two retarded protein complexes were detected, which indicated that the protein factor/s present in intact leaves recognised sequences within the probe. The interaction of both complexes was sequence specific, since addition of an excess of unmarked probe did not prevent the formation of the complexes. On the contrary, when protein extracts were used derived from tomato leaves, 24 hours after inoculation with compatible P.s. tomato one of the two retarded complexes was seriously diminished or became absent, while the other protein-DNA complex remained intact. A similar and reproducible activity of DNA binding to complexes was detected in protein extracts of intact leaves of *Arabidopsis* (Figure 6) with one of the practically annulled complexes when the protein extracts were derived from leaves infected with *P.s. tomato* DC3000 for 24 hours. These results indicate that within the 51 bp defined by the R1 region of the promoter there is an element that acts in cis fundamental for controlling Ep5C expression. In the remaining conditions, when Ep5C is not expressed, this element which acts in cis is specifically joined to protein factors. The formation of this complex is sensitive to bacterial infection, with loss of binding and activation of Ep5C gene occurring simultaneously. These results reveal that the identified complex is acting by repressing Ep5C gene transcription.

### Example 12:

### Generation of transgenic tomato plants with increments and reductions in Ep5C protein level

In order to upregulate *Ep5C* expression, the complete *Ep5C* coding sequence was introduced in the sense direction of transcription downstream with respect to the 35S constitutive promoter of CaMV in order to generate the construction *35S-Ep5Csense* as described above. Similarly, in order to downregulate *Ep5C* expression, a partial sequence of the *Ep5C* gene was introduced in the antisense direction downstream with respect to the 35S promoter of CaMV to generate the 35S-Ep5Cantisense construct. This construction was used for generating transgenic tomato plants by transformation mediated by *Agrobacterium* as has been described previously (Mayda et al., 1999).

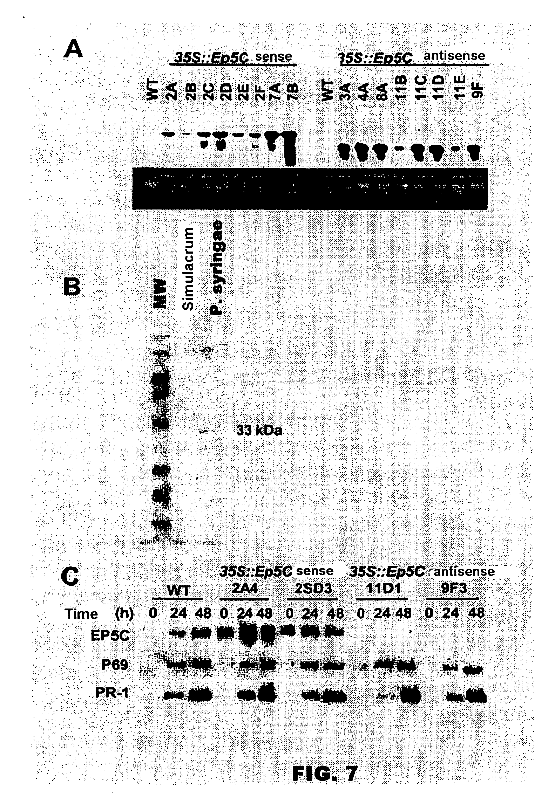

Analysis with transfer of RNA gels was used to detect accumulation of transcripts of the transgenes in various selected T2 transgenic lines and which contained a single insertion of each gene construction. These results, shown in Figure 7A, indicate that both the RNA of transcription sense and the antisense were produced, though with different levels of accumulation, in the selected transformed lines but not in the wild type parental line (WT) without transforming UC82B. In order to complement these studies at the protein level, we generated antibodies against the region of the carboxylic end of Ep5C. To do this, a GST-Ep5C fusion protein was constructed and purified as described above. GST-Ep5C protein was used as immunogen in rabbits to generate polyclonal antibodies. These antibodies were characterised and shown to be specific for the 33 kDa protein which accumulates with detectable levels in tomato leaves infected with *P. syringae* while the same antibody did not recognise any protein in the raw protein extracts from plants in which inoculation was simulated (Figure 7B). Using this specific antibody, we studied the presence of Ep5C protein in raw protein extracts taken from sense and antisense plants. Most of the selected sense lines showed constitutive accumulation of protein Ep5C while those of wild type and all the antisense transgenic lines did not do so. Moreover, neither expression in the normal sense of transcription nor the antisense of *Ep5C* in transgenic tomato plants had any appreciable effect on normal growth and development in successive generations.

As *Ep5C* expression is rapidly induced by infection with *P.s.* tomato in wild type tomato plants, in some of the selected transgenic lines an evaluation was made of the induction of the Ep5C protein accumulation following inoculation with *P.s. tomato.* Here we show the results of two of the sense lines (lines 2A4 and 2D3) and two antisense lines (lines 11D1 and 9F3). Results were compared with wild type plants and also with azygotic plants derived from the initial transgenic plants T1 and in which it had been verified that they did not have any transgene. Western type transfers of the wild type plants showed that Ep5C is absent in untreated plants but accumulates to high levels 24 hours after inoculation with P.s. tomato (Figure 7C). Each of the transgenic lines expressing the construction in the normal sense of transcription (Figure 7C) showed that Ep5C protein accumulates at high levels in untreated plants, and shows slight increases in protein accumulation with respect to its constitutive level following attack by the virulent bacterium. On the contrary, none of the transgenic lines that expressed the antisense construction (results shown in lines 11D1 and 9F3, Figure 7C) showed a detectable level of Ep5C protein, not even after inoculation with P.s. tomato. So, in antisense plants the induced correct accumulation of Ep5C protein is blocked, while sense plants are clear superproducers of that same protein. Moreover, given that accumulation of transcript of the endogenous *Ep5C* gene following induction by infection with P.s. tomato is not affected in the antisense plants, the absence of accumulation of Ep5C protein in the antisense lines is attributable to an inhibition of mRNA translation of the endogenous *Ep5C* by interference with the antisense transcripts. The results therefore indicate that inhibition of the accumulation of Ep5C protein in the antisense plants, or alternatively its constitutive accumulation in the sense plants, is significant and demonstrates the efficiency of the antisense strategy used. Indeed, assays were conducted on protein samples derived from sense and antisense plants in order to detect any alteration in the accumulation of defence-related proteins normally induced by infection with *P.s. tomato* in tomato, such as PR-1 (Tomero et al., 1997) and P69 (Jorda et *al.,* 1999). Analysis by Western type transfer of these two differentiable proteins revealed that neither the sense plants nor the antisense plants showed any major alterations with respect to the wild type plants in their capacity to activate these defences (Figure 7C), thus proving the specificity of the invention.

### Example 13:

### Assay in antisense plants of foliar disease development and resistance to Pseudomonas syringae pv. tomato

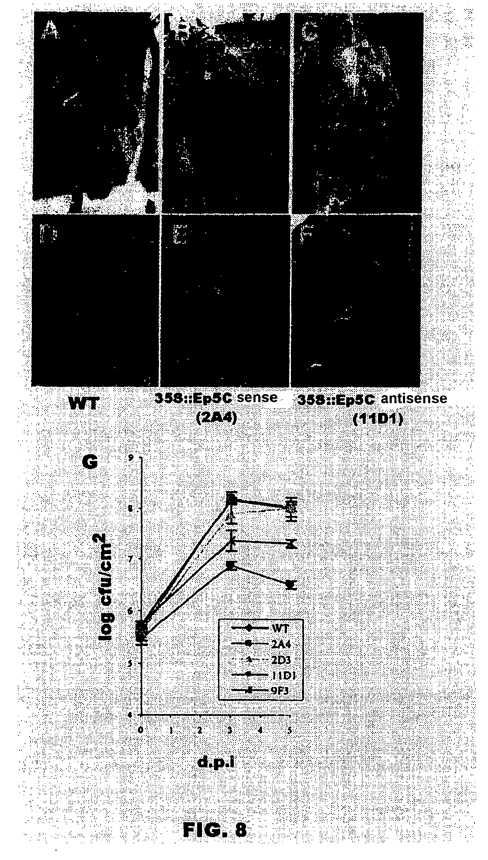

*Ep5C* expression induced by bacteria suggested that this could be important for resistance to the disease. In order to check this, both from the perspective of Ep5C action as a virulence factor, or alternatively, as a component of the defensive arsenal which participates in resistance, we studied the response of transgenic tomato plants to the bacterial pathogen P.s. tomato which causes bacterial spot disease in tomato. The disease is not systemic and so symptoms are developed on leaves inoculated with bacteria. Symptoms of foliar disease include formation of primary necrotic lesions that later on expand and are surrounded by regions of chlorotic tissue. The later symptoms consist of widely extensive necrosis and in the last stage the abscission of the leaves (Jones et *al.,* 1991). To carry out these studies we inoculated different sense and antisense plants and also wild type (WT) with P.s. tomato and we examined the resistance comparing symptoms of the disease and bacterial growth. Figure 8 shows that 7 days after inoculation with P.s. tomato normal lesions of spot disease were detected on the wild type leaves (Figure 8A and Figure 8D) and also on the leaves of plants with an over-accumulation of. Ep5C (Figure 8B and Figure 8E; the results shown here correspond to line 2A4, a representative line). So, there were no visible differences between wild type plants and sense plants in the number and size of lesions that formed in response to bacteria infections.

Unexpectedly, antisense tomato plants were found to behave in the totally opposite fashion. Analysis of the symptoms of various independent lines with *Ep5C* in antisense revealed that the characteristic symptoms of the foliar disease were to a large degree reduced, if not absent, in comparison with those observed in the wild type plants and in lines with an over-accumulation of Ep5C (Figure 8C and Figure 8F; the results shown correspond to the transgenic line 11D1, a representative line). So, the data suggest that induction and correct accumulation of Ep5C are fundamental to the development of symptoms of bacterial spot disease.

Moreover, a correlation was established between the development or absence of development of the symptoms of the disease and the degree of bacterial growth in inoculated leaves. Figure 8G shows that three to five days after inoculation, plants with an over-accumulation of Ep5C (the results shown in Figure 8G represent bacterial growth curves of two of these lines, line 2A4 and line 2D3) behave as wild type plants, neither reducing nor increasing bacterial growth. On the contrary, bacterial growth was drastically reduced in antisense plants where the c.f.u. number was in the range of 10 to 100 times less than in wild type plants. The results of Figure 8G are presented for two of these antisense transgenic lines (for example, 11D1 and 9F3). So, in plants in which Ep5C has been deactivated, resistance to the virulent bacterium *P.s. tomato* is increased simultaneously with a reduction of lesions due to the disease. Moreover, this resistance does not seem to be mediated by a hyperactivation of the SA pathway since expression of response genes to SA *PR1* and P69, measured at the protein level (Figure 7C) or at the mRNA accumulation level (data not shown) was similar in the wild type and in antisense plants. In a similar way, gene *pin-1* expression, used as a marker of the jasmonate pathway, remains in antisense plants as in wild type controls, highlighting the fact that the resistance pathway controlled by jasmonic acid also remains intact in transgenic lines. These results support the fact that the peroxidase enzyme encoded by *Ep5C* gene is a plant factor required for correct growth and virulence of bacteria.

Therefore, the resistance mediated by antisense expression of *Ep5C* represents a new form of resistance to the disease based on the loss of a gene required during compatible interaction between *P. syringae* and the tomato plants and not the activation of known defence pathways of the host.

### Description of the figures:

Figure 1: Expression patterns of Ep5C in tomato plants
   A. comparison with PR-1 and pin-1
   B: With or without avirulence gene avrPto
   C: Distribution on the leaf
Figure 2: Induction of Ep5C H₂O₂
   A. Infiltration of xanthine and xanthine oxidase (X-XO)
   B. Infiltration of glucose and glucose oxidase (G-GO)
   C: Infiltration of xanthine and xanthine oxidase (X-XO) with superoxide dismutase (SOD) or catalase (CAT)
   D. Direct induction with H₂O₂
   E: Blocking of induction by H₂O₂ by means of dimethylthiourea (DMTU) or by means of methyl ester of 2,5-dihydroxycinamic acid (DHC)
Figure 3: Schematic representation of Ep5C gene
Figure 4: Analysis of Ep5C expression in transgenic plants of Arabidopsis
   A: Inoculation with P. syringae (P.s.t. DC3000)
      Inoculation with P. syringae (P.s.t. DC3000) carrier of avirulence gene
      AvrRpm1
      Simulated inoculation (control)
   B: Induction with H₂O₂
   C. Infiltration of xanthine and xanthine oxidase (X-XO)
      Infiltration of glucose and glucose oxidase (G-GO)
      Infiltration of xanthine and xanthine oxidase (X-XO) with superoxide
      dismutase (SOD) or catalase (CAT)
      Direct induction with H₂O₂
      Blocking of induction by H₂O₂ by means of dimethylthiourea (DMTU) or
      by means of methyl ester of 2,5-dihydroxycinamic acid (DHC)
Figure 5: Unidirectional deletions starting from the 5' end
Figure 6: R1 probe incubation with extracts of control leaves from tomato plants or Arabidopsis
Figure 7: Accumulation of transgene transcripts in T2 transgenic plants
   A: By means of gel transfer analysis
   B: Immunoelectrophoresis using polyclonal Ab against Ep5C protein (dd kDa)
   C: Immunoelectrophoresis using polyclonal Ab against Ep5C protein (dd kDa) in wild plants (WT) or sense or antisense transgenic plants
Figure 8: Assay in transgenic plants of resistance to foliar disease due to Pseudomonas syringae pv. tomato
   A-D: Wild (WT)
   B-E: Sense transgenic plant (2A4)
   C-F: Antisense transgenic plant (11D1)
   G: Bacterial growth: Ordinates: colony forming units (cfu x cm²); Abscissa: days per inoculation (d.p.i.)

## Claims

1. Transgenic plants resistant to Pseudomonas syringae pv. tomato **characterised by** presenting an inhibition of inducible expression of the Ep5C gene.

2. Transgenic plants according to claim 1, **characterised in that** the inhibition of the inducible expression of the Ep5C gene consists of said plants having at least one part of.the Ep5C gene sequence in antisense.

3. Transgenic plants according to either of claims 1 or 2, **characterised in that** the antisense sequence corresponds to a segment of 309 nucleotides of the coding sequence of the Ep5C gene, starting from its 3' end.

4. Transgenic plants according to any of claims 1 to 3, **characterised by** being tomato plants (genus Lycopersicon).

5. Procedure for obtaining transgenic plants resistant to Pseudomonas syringae pv. tomato which comprises:
i) Amplifying by PCR the fragment of DNA closest to the 3' end of pCEVI16;
ii) Subcloning the amplified fragment in a binary vector under control of a promoter, in the antisense direction;
iii) Transferring the gene constructions obtained to a microorganism capable of transforming plants;
iv) Transforming said plants with the micoorganism containing the antisense gene constructions for the Ep5C gene.

6. Procedure according to claim 5, **characterised in that** the DNA fragment that is amplified in stage i) has 309 bases.

7. Procedure according to either of claims 5 or 6, **characterised in that** the subcloning in antisense direction of stage ii) is done under control of the constitutive promoter fragment 35S of CaMV.

8. Procedure according to any of claims 5 to 7, **characterised in that** the gene constructions obtained are transferred in stage iii) to Agrobacterium tumefaciens.

9. Procedure according to any of claims 5 to 8, **characterised in that** the transformed plants belong to the genus Lycopersicon (tomato).

10. Use of the promoter included in SEQ ID NO:1, in the control of gene expression in plants.

11. Use according to claim 10, **characterised in that** at least the sequence region of the promoter included between positions 320 to 378 of SEQ ID NO:1 is used.

12. Use according to claim 11, **characterised in that** at least a region R1 of 51 bp included between positions 320 to 370 of SEQ ID NO:1 corresponding to the promoter is used, which comprises at least 3 sequence segments which act in cis, selected from among the following positions: MYB Box (335 to 343 of SEQ ID NO1), H Box (344 to 349 of SEQ ID NO1 and G Box (354 to 359 of SEQ ID NO1).
